# EUROPÄISCHE PATENTANMELDUNG

(11) **EP 0 716 087 A1**
(43) Veröffentlichungstag der Anmeldung: **12.06.1996**
(21) Anmeldenummer: 95118497.7
(22) Anmeldetag: 24.11.1995
(51) Int. Cl.: C07D 333/36, C07D 277/46, C07D 285/12, C07D 409/12

(54) **Heterocyclische Amide zur Beeinflussung der Kalium-Kanäle**

(30) Priorität: 05.12.1994 AT 2252/94
(71) Anmelder: Hafslund Nycomed Pharma AG, A-4020 Linz (AT)
(72) Erfinder: Esch, Peter, Dr., A-4020 Linz (AT); Towart, Robertson, Dr., A-4061 Pasching (AT); Rovenszky, Franz, Dipl.-Ing. Dr., A-4020 Linz (AT)
(74) Vertreter: Landgraf, Elvira

(57) **Zusammenfassung**

Die Erfindung betrifft heterocyclische Amide der Formel 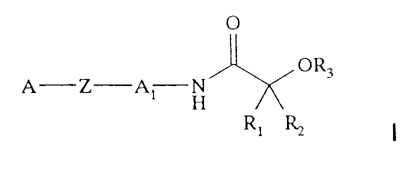
in der die Reste A, Z, A₁, R₁, R₂ und R₃ die in der Beschreibung angegebene Bedeutung haben, ein Verfahren zur Herstellung dieser Amide und ihre Verwendung als Wirkstoffe zur Heilung und Linderung von Störungen und Krankheiten, die durch die Beeinflussung von Kaliumkanälen behandelt werden können.

## Beschreibung

Die Erfindung betrifft neue heterocyclische Amide, die als Wirkstoffe gegen Störungen und Krankheiten, die durch Beeinflussung von Kalium-Kanälen geheilt oder verbessert werden, eingesetzt werden können.

Es ist bekannt, daß unkontrollierte oder instabile Muskelspasmen der Harnblasenmuskulatur Harninkontinenz erzeugen können. Obwohl der Parasympathikus eine große Rolle in der kontrollierten, physiologischen Funktion der Harnblase beim Menschen spielt, sind unkontrollierte Harnabgaben doch häufiger auf eine Überempfindlichkeit der glatten Harnblasenmuskulatur zurückzuführen (K.-E. Andersson, Pharmacology of lower urinary tract smooth muscles and penile erectile tissue, Pharmacol. Rev., 45, 253-308, 1993). Gewisse Medikamente, z.B. Spasmolytika, Kalziumantagonisten und Kaliumkanalaktivatoren, sind in der Lage, diese Überempfindlichkeit zu dämpfen. Einige davon werden schon in der Praxis als Prophylaktika gegen Harninkontinenz angewendet (K-E. Andersson, Current concepts in the treatment of disorders of micturition, Drugs, *35*, 477-494, 1988). Der Nachteil all dieser dort beschriebenen Verbindungen ist ihr ausgeprägtes Nebenwirkungsprofil, das unter anderem kardiovaskuläre und gastrointestinale Störungen beinhaltet (ibid.).

Aus EP-A 0 524 781 sind substituierte Amide bekannt, die die Öffnung von Kaliumkanälen bewirken. Diese substituierten Amide enthalten als Substituenten einen gegebenenfalls substituierten Phenyl- oder Pyridylrest. Aus EP-A 0 617 010 sind ähnlich wirksame Amide bekannt, die als Substituenten einen gegebenenfalls substituierten Phenyl-, Pyridyl-, Pyrimidyl-, Pyridazinyl- oder Pyrazinylrest enthalten.

Aufgabe der vorliegenden Erfindung war es, neue Verbindungen bereitzustellen, die eine ausgezeichnete Wirksamkeit bei der Beeinflussung von Kaliumkanälen und hohe Selektivität bei verminderten Nebenwirkungen aufweisen und dadurch den bekannten Verbindungen überlegen sind.

Unerwarteterweise konnte diese Aufgabe durch die Bereitstellung neuer heterocyclischer Amide gelöst werden, die als heteroaromatischen Substituenten einen Schwefel oder einen Schwefel und Stickstoff enthaltenden Ring aufweisen.

Gegenstand der Erfindung sind demnach heterocyclische Amide der Formel I 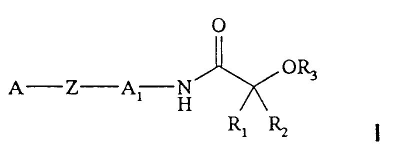
in welcher
A einen aromatischen oder S- oder N- heteroaromatischen Ring mit 5 - 10 C-Atomen, der gegebenfalls ein- oder mehrfach durch geradkettiges oder verzweigtes C₁-C₄ Alkyl, C₁-C₄ Alkoxy, Hydroxy Halogen oder CF₃, CF₃CF₂ substituiert sein kann,
Z die Reste C=O, SO oder SO₂,
A₁ ein heteroaromatisches System der Formel IIa-IIc 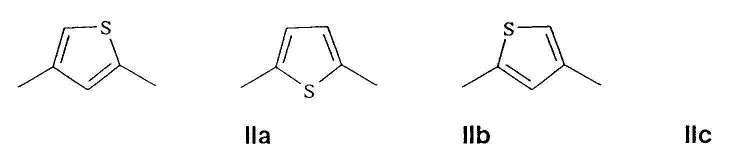
oder ein heteroaromatisches System der Formel IIIa-IIIc 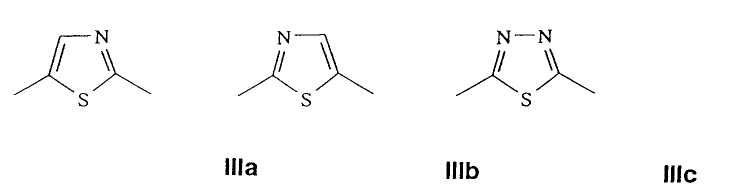
R₁ und R₂ unabhängig voneinander H, geradkettiges oder verzweigtes (C₁-C₄)-Alkyl oder fluoriertes Alkyl,
und R₃ H, geradkettiges oder verzweigtes (C₁-C₄)-Alkyl oder eine unter physiologisches Bedingungen abspaltbare Gruppe bedeutet.

Die Verbindungen der Formel I können als Mischung ihrer optischen Antipoden, aber auch jeweils in enantiomerenreiner Form vorliegen. Sowohl Mischungen der optischen Antipoden der Verbindungen der Formel I als auch die jeweils enantiomerenreine Form sind Gegenstand der vorliegenden Erfindung.

In der Formel I bedeutet der Rest A einen aromatischen oder S- oder N- heteroaromatischen Ring mit 5 - 10 C-Atomen, beispielsweise einen Phenylrest, einen Thienylrest, einen Pyridylrest, einen Pyrimidylrest, einen Pyridazylrest, einen Imidazolylrest. Diese Reste können gegebenenfalls ein -oder mehrfach substituiert sein, beispielsweise durch geradkettiges oder verzweigtes C₁-C₄ Alkyl, wie Methyl, Ethyl, Propyl, Isopropyl, Butyl, Isobutyl oder t-Butyl oder durch geradkettiges oder verzweigtes C₁-C₄ Alkoxy, beispielsweise Methoxy, Ethoxy, Propoxy oder Butoxyreste. Weiters kann dieser heteroaromatische Ring ein- oder mehrfach durch Hydroxy, Halogen, wie Chlor, Fluor oder Brom oder durch die Reste CF₃, CF₃CF₂ substituiert sein.

Bevorzugt sind jene Verbindungen, in denen der Rest A Phenyl, durch Halogen, vorzugsweise Chlor oder Fluor oder durch CF₃, CF₃CF₂ einfach substituiertes Phenyl oder 2- oder 3- Thienyl bedeutet.

Besonders bevorzugt sind Verbindungen, in denen der Rest A Phenyl, das gegebenenfalls durch Halogen substituiert ist, bedeutet.

Der Rest Z bedeutet eine Carbonylgruppe C=O, eine Sulfinylgruppe SO, oder eine Sulfonylgruppe SO₂.

Bevorzugt sind Verbindungen, in denen der Rest Z eine Carbonylgruppe oder eine Sulfonylgruppe bedeutet.

Der Rest A₁ bedeutet ein heteroaromatisches System der Formeln IIa - IIc 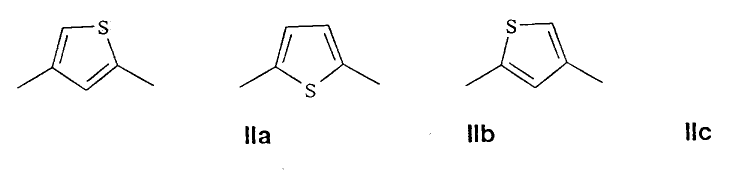
oder ein heteroaromatisches System der Formel IIIa-IIIc 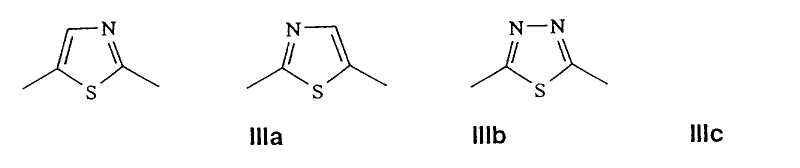
Bevorzugt sind Verbindungen, in denen der Rest A₁ ein heteroaromatisches System der Formel IIb, IIIb oder IIIc bedeutet.

Besonders bevorzugt sind Verbindungen, in denen der Rest A₁ ein heteroaromatisches System der Formel IIb bedeutet.

Die Reste R₁ und R₂ bedeuten unabhängig voneinander H, einen geradkettigen oder verzweigten C₁-C₄-Alkylrest, wie beispielsweise einen Methyl-, Ethyl-Propyl-, Isopropyl-, Butyl-, Isobutyl- oder t-Butylrest oder fluorierten Alkylrest, beispielsweise den Rest CF₃ oder CF₃CF₂.

Bevorzugt sind jene Verbindungen, in denen die Reste R₁ und R₂ unabhängig voneinander einen geradkettigen oder verzweigten C₁-C₄-Alkylrest oder den Rest CF₃ bedeuten.

Besonders bevorzugt sind Verbindungen, in denen die Reste R₁ und R₂ unabhängig voneinander einen Methylrest oder den Rest CF₃ bedeuten.

Der Rest R₃ bedeutet H, geradkettiges oder verzweigtes Alkyl mit 1 -4 C- Atomen, einen Methyl-, Ethyl-, Propyl-, Isopropyl-, Butyl-, Isobutyl- oder t-Butylrest oder eine unter physiologischen Bedingungen abspaltbare Gruppe. Solche unter physiologischen Bedingungen abspaltbare Gruppen sind beispielsweise der Rest CON, Acetyl- oder Acetylalkylreste.

Ein weiterer Gegenstand der Erfindung ist ein Verfahren zur Herstellung von Verbindungen der Formel I 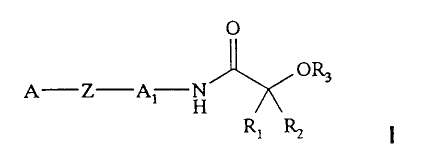
indem man eine Verbindung der Formel IV

A-Z-A₁-NH₂ IV

mit Hilfe bekannter Kopplungsreagenzien mit einer Verbindung der Formel V 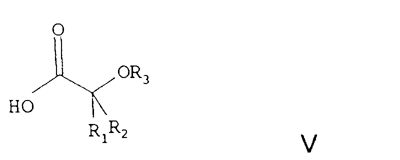
koppelt und gegebenfalls die so erhaltene Mischung der optischen Antipoden in die Enantiomeren trennt.
Die Verbindungen der Formel V können auch in einer ihrer optisch aktiven Formen eingesetzt werden, wobei als Produkt jeweils die entantiomerenreine Form einer Verbindung der Formel I erhalten wird.

Verbindungen der Formel IV und V sind literaturbekannt oder können nach dem Fachmann geläufigen Verfahren hergestellt werden.

Als Kopplungsreagentien werden beispielsweise aus der Peptidsynthese bekannte Kopplungsreagentien, beispielsweise SOCl₂, DCC, N,N-Carbonyldiimidazol und dergleichen verwendet.

Zur Durchführung des Verfahrens wird eine Verbindung der Formel IV in einem geeigneten Verdünnungsmitttel mit einem Kopplungsreagenz, beispielsweise Thionylchlorid, DCC oder N,N-Carbonyldiimidazol in Kontakt gebracht. Als Verdünnungsmittel kommen unter Reaktionsbedingungen inerte Verdünnungs- oder Lösungsmittel in Frage, beispielsweise N,N-Dimethylacetamid, Dimethylformamid oder Tetrahydrofuran und dergleichen. Die Reaktionstemperatur ist von der Wahl des Kopplungsmittels abhängig und kann zwischen - 25°C und 100 °C variieren.
Anschließend wird eine Verbindung der Formel V zugegeben und bei einer Temperatur von -25°C bis 100°C, vorzugsweise bei Raumtemperatur reagieren gelassen.
Das erhaltene Reaktionsprodukt wird anschließend durch Extraktion und Abziehen des Lösungsmittels isoliert und kann gegebenenfalls durch bekannte Methoden, beispielsweise Umkristallisieren oder durch Chromatographie weiter gereinigt werden.

Die Auftrennung der gegebenenfalls in Form einer Mischung der optischen Antipoden vorliegenden Reaktionsprodukte erfolgt nach dem Fachmann geläufigen Methoden, beispielsweise durch Kopplung mit (1S)-(-) Camphansäurechlord oder mit (R)-(+)-Methylbenzylisocyanat, worauf die so erhaltenen Diastereomeren nach üblichen Methoden, beispielsweise durch Kristallisieren oder Chromatographie getrennt werden und die Schutzgruppen abgespalten werden

Die erfindungsgemäßen Verbindungen weisen ausgezeichnete Wirksamkeit bei der Beeinflussung von Kaliumkanälen bei verminderten Nebenwirkungen auf. Sie zeigen hohe Selektivität bei Störungen und Erkrankungen der Blase und der ableitenden Harnwege, die durch Beeinflussung von Kaliumkanälen geheilt oder gelindert werden.

Aufgrund dieser pharmakologischen Eigenschaften können die neuen Verbindungen allein oder in Mischung mit anderen Wirksubstanzen in Form üblicher galenischer Zubereitungen als Heilmittel zur Behandlung von Störungen oder Erkrankungen, die durch Beeinflussung der Kaliumkanäle geheilt oder gelindert werden, Verwendung finden.

Die Erfindung bezieht sich weiterhin auf Heilmittel, die z.B. in Form pharmazeutischer Präparate Verwendung finden, welche die erfindungsgemäßen Verbindungen der Formel (I) und ihre Salze in Mischung mit einem für die orale, enterale, parenterale und topicale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline und dergleichen enthalten.

Die pharmazeutischen Präparate können in fester Form, z.B. als Tabletten, Filmtabletten, Dragees, Suppositorien, Kapseln, Mikrokapseln oder in flüssiger Form, z.B. als Lösungen, Injektionslösungen, Suspensionen oder Emulsionen oder in Zusammensetzungen mit verzögerter Freigabe des Wirkstoffes vorliegen.
Gegebenenfalls können sie sterilisiert werden und/oder sie enthalten Hilfsstoffe wie Konservierungs-, Stabilisierungs- oder Emulgiermittel, Salze zur Veränderung des osmotischen Drucks oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben genannten Hilfs- und/oder Trägerstoffen zu Kombinationspräparaten formuliert werden.

Die neuen Verbindungen können in den erfindungsgemäßen Zusammensetzungen in einem Anteil von 0,1-200 mg pro Tablette vorhanden sein, wobei der Rest ein pharmazeutisch annehmbarer Füllstoff ist.

Eine geeignete Dosis zur Verabreichung der Verbindungen beträgt etwa 0,01-100 mg/kg pro Tag, jedoch kommen, je nach Zustand des zu behandelnden Patienten auch andere Dosen in Frage. Die neuen Verbindungen können auch in mehreren Dosen verabreicht werden.

### Beispiel 1: N-[5-(Phenylcarbonyl)-thien-2-yl]-3,3,3-trifluor-2-hydroxy-2-methylpropanamid

Zu 3,3,3-Trifluor-2-hydroxy-2-methylpropansäure (240 mg, 1,52 mMol) in 5 ml *N*,*N*-dimethylacetamid werden bei -15/-10 °C Thionylchlorid (110 µl, 1,51 mMol) zugegeben und bei dieser Temperatur für eine Stunde nachgerührt. Anschließend wird 2-Amino-5-(phenylcarbonyl)-thiophen (200 mg, 0,983 mMol) zugegeben und das Reaktionsgemisch auf Raumtemperatur angeglichen. Nach beendeter Reaktion wird das Reaktionsgemisch auf 200 ml Wasser gegossen und mit 3 x 100 ml Ethylacetat extrahiert. Die vereinigten organischen Phasen werden über Magnesiumsulfat getrocknet, filtriert und das Lösungsmittel abdestilliert. Das verbleibende orangefarbige Öl wird mittels Flash-chromatographie (Eluens: 50% v/v Ethylacetat in Petrolether (40/60)) gereinigt.
Ausbeute 290 mg *N*-[5-(Phenylcarbonyl)-thien-2-yl]-3,3,3-trifluor-2-hydroxy-2-methylpropanamid (86% d. Th) als leichtgelbe Kristalle.
Fp: 169-170 °C.
¹H NMR (400 MHz, CDCl₃) 1.74 (s, 3 H), 6.89 (d, *J* = 4.0 Hz, 1 H), 7.41-7.46 (m, 2 H), 7.48 (d, *J* = 4.0 Hz, 1 H), 7.51-7.56 (m, 1 H), 7.73-7.78 (m, 2 H), 10.02 (s, 1 H). ¹³C NMR (100 MHz, CDCl₃) 20.0, 75.6 (q, ²*J*_{C,F} = 30.2 Hz), 114.5, 123.9 (q, ¹*J*_{C,F} = 285.8 Hz), 128.5, 129.0, 132.2, 134.6, 134.7, 138.0, 147.3, 166.3, 189.5.

Die Synthese von 3,3,3-Trifluor-2-hydroxy-2-methylpropansäure wird beschrieben von R.A. Darrall, F. Smith, M. Stacey und J.C. Tatlow J. Chem. Soc. 1951, 2329.

Das Ausgangsprodukt wird folgendermaßen hergestellt:
5-(Phenylcarbonyl)-thiophen-2-carbonsäure:
Zu Diisopropylamin (11,6 ml, 82,2 mMol) in 150 ml THF abs. wird bei -70 °C eine 1,6 M Lösung von n-Butyllithium in n-Hexan (51,0 ml, 81,3 mMol) zugetropft. Nach 15 min. Rühren bei -70 °C wird Thiophen-2-carbonsäure (5,00 g, 39,0 mMol) zugegeben und das Gemisch bei -70 °C gehalten. Nach 45 min. wird Benzonitril (5,0 ml, 49 mMol) zugetropft und 90 min bei -70 °C gerührt. Nach Angleichung an Raumtemperatur wird das Reaktionsgemisch auf 200 ml Wasser gegossen und mit 200 ml Ether extrahiert und diese organische Phase wird verworfen. Die wäßrige Phase wird mit einer 6 N HCl-Lösung auf pH 2 eingestellt und mit 2 x 200 ml Ether extrahiert. Die vereinigten organischen Phasen werden mit Magnesiumsulfat getrocknet und das Lösungsmittel abdestilliert. Der Rückstand wird in 50 ml einer 6 N HCl-Lösung suspendiert und eine Stunde auf 80 °C erhitzt. Die ausgefallenen Kristalle werden abfiltriert, unter Vakuum über Nacht getrocknet.
Ausbeute: 8,3 g 5-(Phenylcarbonyl)-thiophen-2-carbonsäure (92% d. Th) als leichtgelbe Kristalle.
Fp: 176-177 °C.
¹H NMR (400 MHz, DMSO-*d₆*) 7.55-7.63 (m, 2 H), 7.68-7.75 (m, 1 H), 7.70 (d, *J* = 3.9 Hz, 1 H), 7.77 (d, *J* = 3.9 Hz, 1 H), 7.83-7.90 (m, 2 H). ¹³C NMR (100 MHz, DMSO-*d₆*) 128.9, 129.1, 133.2, 133.5, 135.2, 136.8, 141.4, 146.8, 162.5, 187.7.

[5-(Phenylcarbonyl)-thien-2-yl]-carbamidsäure tert.-butyl ester:
Zu 5-(Phenylcarbonyl)-thiophen-2-carbonsäure (0,99 g, 4,3 mMol), in 40 ml tert.-Butanol abs. werden Triethylamin (0,72 ml, 5,2 mMol) und Diphenylphosphorylazid (1,1 ml, 5,1 mMol) zugegeben. Das Reaktionsgemisch wird für 5 Stunden unter Rückfluß gekocht. Das Lösungsmittel wird abdestilliert. Der Rückstand wird aufgenommen in Ethylacetat und mit Wasser gewaschen. Die organische Phase wird über Magnesiumsulfat getrocknet, filtriert und das Lösungsmittel abdestilliert. Der verbleibende Feststoff wird mittels Flash-chromatographie (Eluens: 25% v/v Ethylacetat in Petrolether (40/60)) gereinigt.
Ausbeute: 400 mg [5-(Phenylcarbonyl)-thien-2-yl]-carbamidsäure tert.-butyl ester, (31% d. Th) als orangefarbige Kristalle.
Fp.: 185-186 °C.
¹H NMR (400 MHz, CDCl₃) 1.54 (s, 9 H), 6.58 (d, *J* = 4.2 Hz, 1 H), 7.43 (d, *J* = 4.2 Hz, 1 H), 7.43-7.48 (m, 2 H), 7.52 (bs, 1 H), 7.50-7.55 (m, 1 H), 7.78-7.82 (m, 2 H). ¹³C NMR (100 MHz, CDCl₃) 28.2, 82.7, 111.1, 128.3, 128.9, 131.6, 133.6, 134.5, 138.6, 150.1, 151.6, 188.1.

2-Amino-5-(phenylcarbonyl)-thiophen
[5-(Phenylcarbonyl)-thien-2-yl]-carbamidsäure tert.-butyl ester (0,30 g, 0.99 mMol) wird in 5 ml Trifluoressigsäure gelöst und für 2 Stunden bei Raumtemperatur gerührt. Die Trifluoressigsäure wird abdestilliert und der Rückstand wird zwischen einer halbgesättigten Natriumhydrogencarbonat-Lösung und Dichlormethan verteilt. Die Phasen werden getrennt und die wäßrige Phase mit Dichlormethan extrahiert. Die vereinigten organischen Phasen werden mit Kaliumcarbonat getrocknet, filtriert und das Lösungsmittel abdestilliert.
Ausbeute: 0,20 g 2-Amino-5-(phenylcarbonyl)-thiophen (99% d. Th) als rote Kristalle.
¹H NMR (400 MHz, DMSO-*d₆*) 5.99 (d, *J* = 4.3 Hz, 1 H), 7.18 (bs, 2 H), 7.23 (d, *J* = 4.3 Hz, 1 H), 7.45-7.60 (m, 3 H), 7.62-7.67 (m, 2 H).

### Beispiel 2: N-[5-(Phenylcarbonyl)-thiazol-2-yl]-3,3,3-trifluor-2-hydroxy-2-methylpropanamid

Zu 3,3,3-Trifluor-2-hydroxy-2-methylpropansäure (150 mg, 0,949 mMol) in 5 ml *N*,*N*-dimethylacetamid werden bei -15/-10 °C Thionylchlorid (80 µl, 1,1 mMol) zugegeben und bei dieser Temperatur für eine Stunde nachgerührt. Nach Zugabe von 2-Amino-5-(phenylcarbonyl)-thiazol (130 mg, 0,636 mMol) und Angleichung an Raumtemperatur wird das Reaktionsgemisch 2,5 Stunden gerührt, anschließend auf 100 ml Wasser gegossen und mit 3 x 100 ml Ethylacetat extrahiert. Die vereinigten organischen Phasen werden mit 50 ml einer gesättigten Natriumchlorid-Lösung gewaschen, über Magnesiumsulfat getrocknet, filtriert und das Lösungsmittel abdestilliert. Das verbleibende Öl wird mittels Flash-chromatographie (Eluens: 33% v/v Ethylacetat in Petrolether (40/60)) gereinigt und ergibt *N*-[5-(Phenylcarbonyl)-thiazol-2-yl]-3,3,3-trifluor-2-hydroxy-2-methylpropanamid (0,10 g, 46%) als leichtgelbe Kristalle. Diese Kristalle werden in Dichlormethan digeriert.
Ausbeute: 50 mg der Titelverbindung als weiße Kristalle.
Fp.: 193-194 °C.
¹H NMR (400 MHz, DMSO-*d₆*) 1.62 (s, 3 H), 7.56-7.61 (m, 3 H), 7.67-7.72 (m, 1 H), 7.84-7.88 (m, 2H), 8.17 (s, 1 H).

Das Ausgangsprodukt wird folgendermaßen hergestellt:
Thiazol-2-ylcarbamidsäure-tert.-butylester
2-Aminothiazol (5,00 g, 49,9 mMol) und di-tert.-butylpyrocarbonat (23,0 ml, 100 mMol) in 250 ml Methanol abs. werden 2 Stunden unter Rückfluß gekocht. Zusätzlich wird di-tert.-butylpyrocarbonat (11,5 ml, 50,1 mMol) zugetropft und das Gemisch für weitere 2 Stunden unter Rückfluß gekocht. Das Reaktionsgemisch wird in 250 ml Methanol aufgenommen und mit Aktivkohle verrührt, filtriert und die Aktivkohle mit Dichlormethan gewaschen. Nach Verdampfen der Lösungsmitteln der vereinten organischen Phasen wird der Rückstand mit Hexan digeriert.
Ausbeute: 9,41 g Thiazol-2-ylcarbamatesäure-tert.-butylester (94% d. Th.) als hellgelbe Kristalle.
Fp: 178-179 °C (Hexan).
¹H NMR (400 MHz, DMSO-*d₆*) 1.48 (s, 9 H), 7.13 (d, *J =* 3.8 Hz, 1 H), 7.35 (d, *J* = 3.8 Hz, 1 H). ¹³C NMR (100 MHz, DMSO-*d₆*) 28.0, 81.1, 112.9, 137.9, 152.9, 159.9.

[5-(Phenylcarbonyl)-thiazol-2-yl]carbamidsäure tert.-butyl ester
Zu Diisopropylamin (1,55 ml, 11,0 mmol) in 100 ml THF abs. wird bei -70 °C eine 1,6 M Lösung von n-Butyllithium in n-Hexan (6,90 ml, 11,0 mMol) zugetropft. Nach 30 min. Rühren bei -70 °C wird Thiazol-2-ylcarbamidsäure-tert.-butylester (1,00 g, 4,99 mMol) zugegeben und das Gemisch bei -70 °C gehalten. Nach 60 min. wird Benzonitril (0,60 ml, 5,9 mMol) zugetropft und 60 min. bei -70 °C gerührt. Nach Angleichung des Reaktionsgemisches an Raumtemperatur wird für 2 Stunden gerührt. Das Reaktionsgemisch wird auf 300 ml halbgesättigte Ammoniumchlorid-Lösung gegossen und mit 4 x 100 ml Dichlormethan extrahiert. Die vereinigten organischen Phasen werden über Magnesiumsulfat getrocknet, filtriert und das Lösungsmittel abdestilliert. Das Rohprodukt (1,40 g) wird in 150 ml Ether suspendiert und 100 ml 0.1 N HCl-Lösung wird zugegeben. Das Gemisch wird für 1,5 Stunden unter Rückfluß gekocht. Das Reaktionsgemisch wird in 100 ml 0,1 N HCl-Lösung aufgenommen und mit 4 x 100 ml Dichlormethan extrahiert. Die vereinigten organischen Phasen werden über Magnesiumsulfat getrocknet, filtriert und das Lösungsmittel abdestilliert.
Ausbeute: 1,15 g [5-(Phenylcarbonyl)-thiazol-2-yl]carbamidsäure tert.-butyl ester (76% d. Th) als gelbe Kristalle.
Fp: 232-233 °C (Zers.).
¹H NMR (400 MHz, CDCl₃ + 10% DMSO-*d₆*) 1.36 (s, 9 H), 7.26-7.32 (m, 2 H), 7.36-7.41 (m, 1 H), 7.58-7.63 (m, 2 H), 7.68 (s, 1 H), 11.26 (bs, 1 H). ¹³C NMR (100 MHz, CDCl₃ + 10% DMSO-*d₆*) 28.1, 82.7, 128.4, 128.5, 131.8, 132.1, 138.1, 146.7, 152.5, 166.6, 187.4.

2-Amino-5-(phenylcarbonyl)-thiazol
[5-(Phenylcarbonyl)-thiazol-2-yl]-carbamidsäure tert.-butyl ester (0,82 g, 2,70 mMol) wird in 25 ml Trifluoressigsäure gelöst und für 1,5 Stunde bei Raumtemperatur gerührt. Die Trifluoressigsäure wird abdestilliert und der Rückstand wird zwischen einer halbgesättigte Natriumhydrogencarbonat-Lösung und Dichlormethan verteilt. Die Phasen werden getrennt und die wäßrige Phase mit Dichlormethan extrahiert. Die vereinigten organischen Phasen werden gewaschene mit einer halbgesättigte Natriumhydrogencarbonat-Lösung über Kaliumcarbonat getrocknet, filtriert und das Lösungsmittel abdestilliert.
Ausbeute: 0,37 g 2-Amino-5-(phenylcarbonyl)-thiazol (67% d. Th) als gelbe Kristalle.
Fp: 147-149 °C.
¹H NMR (400 MHz, DMSO-*d₆*) 7.48-7.53 (m, 2 H), 7.57-7.62 (m, 1 H), 7.62 (s, 1 H), 7.68-7.73 (m, 2 H). ¹³C NMR (100 MHz, DMSO-*d₆*) 127.1, 128.3, 128.7, 131.7, 138.4, 151.0, 174.9, 185.5.

### Beispiel 3: N-[5-(Phenylcarbonyl)-1,3,4-thiadiazol-2-yl]-3,3,3-trifluor-2-hydroxy-2-methylpropanamid

Zu 3,3,3-Trifluor-2-hydroxy-2-methylpropansäure (157 mg, 0,993 mMol) in 5,0 ml *N*,*N*-dimethylacetamid werden bei -20/-15 °C Thionylchlorid (80 µl, 1,1 mMol) zugegeben und bei -15/-10 °C für eine Stunde nachgerührt. Nach Zugabe von 2-Amino-5-(phenylcarbonyl)-1,3,4-thiadiazol (127,7 mg, 0,622 mMol) und Angleichung an Raumtemperatur wirdnach beendeter Reaktion das Reaktionsgemisch auf 80 ml Wasser gegossen und mit 3 x 25 ml Ethylacetat extrahiert. Die vereinigten organischen Phasen werden über Magnesiumsulfat getrocknet, filtriert und das Lösungsmittel abdestilliert. Das verbleibende Öl wird mittels Flash-chromatographie (Eluens: 50% v/v Ethylacetat in Petrolether (40/60)) gereinigt.
Ausbeute: 160 mg *N*-[5-(Phenylcarbonyl)-1,3,4-thiadiazol-2-yl]-3,3,3-trifluor-2-hydroxy-2-methylpropanamid (75% d. Th) als beige Kristalle.
Fp.: 182-183 °C.
¹H NMR (400 MHz, DMSO-*d₆*) 1.65 (s, 3 H), 7.60-7.65 (m, 2 H), 7.62 (bs, 1 H), 7.73-7.78 (m, 1 H), 8.33-8.37 (m, 2 H), 10.8 (bs, 1 H). ¹³C NMR (100 MHz, DMSO-*d₆*) 19.8, 75.3 (q, ²*J*_{C,F} = 29.2 Hz), 125.9 (q, ¹*J*_{C,F} = 285.8 Hz), 128.8, 130.7, 134.3, 134.9, 161.9, 163.6, 169.4, 184.2.

Die Synthese von 2-Amino-5-(phenylcarbonyl)-1,3,4-thiadiazol wird beschrieben von G. Werber, F. Buccheri und M.L. Marino *J*. *Heterocyclic Chem*. **1975**, *12*, 581.

### Beispiel 4: N-[5-(Phenylsulfonyl)-thien-2-yl]-3,3,3-trifluor-2-hydroxy-2-methylpropanamid

Zu 3,3,3-Trifluor-2-hydroxy-2-methylpropansäure (170 mg, 1,08 mMol) in 5 ml *N*,*N*-dimethylacetamid werden bei -15/-10 °C Thionylchlorid (80 µl, 1,1 mMol) zugegeben und bei dieser Temperatur für eine Stunde nachgerührt. Nach Zugabe von 2-Amino-5-(phenylsulfonyl)-thiophen (170 mg, 0,710 mMol) und Angleichung an Raumtemperatur wird das Reaktionsgemisch nach beendeter Reaktion auf 200 ml Wasser gegossen und mit 3 x 100 ml Ethylacetat extrahiert. Die vereinigten organischen Phasen werden über Magnesiumsulfat getrocknet, filtriert und das Lösungsmittel abdestilliert. Das verbleibende orangefarbige Öl wird mittels Flash-chromatographie (Eluens: 50% v/v Ethylacetat in Petrolether (40/60)) gereinigt.
Ausbeute: 202 mg *N*-[5-(Phenylsulfonyl)-thien-2-yl]-3,3,3-trifluor-2-hydroxy-2-methylpropanamid (75% d. Th) als graue Kristalle.
Fp.: 161-163 °C.
¹H NMR (400 MHz, DMSO-*d₆*) 1.57 (s, 3 H), 7.11 (d, *J* = 4.2 Hz, 1 H), 7.58-7.70 (m, 5 H), 7.90-7.93 (m, 2 H).¹³C NMR (100 MHz, DMSO-*d₆*) 19.9, 75.3 (q, ²*J*_{C,F} = 29.2 Hz), 114.3, 125.9 (q, ¹*J*_{C,F} = 285.8 Hz), 126.6, 129.8, 131.4, 132.5, 133.5, 142.6, 147.1, 167.3.

Das Ausgangsprodukt wird folgendermaßen hergestellt:
5-(Phenylsulfonyl)-thiophen-2-carbonsäure
Zu Diisopropylamin (11,6 ml, 82,2 mmol) in 150 ml THF abs. wird bei -78 °C eine 1,6 M Lösung von n-Butyllithium in n-Hexan (51,0 ml, 81,3 mMol) zugetropft. Nach 30 min. Rühren bei -70 °C wird Thiophen-2-carbonsäure (5,00 g, 39,0 mMol) zugegeben und das Gemisch bei -78 °C gehalten. Nach 60 min. wird Diphenyldisulfid (9,39 g, 43,0 mMol) zugegeben und 60 min bei -78 °C gerührt. Nach Angleichung des Reaktionsgemisches an Raumtemperatur wird es auf 400 ml 1 N HCL-Lösung gegossen und mit 3 x 200 ml Dichlormethan extrahiert. Die vereinigten organischen Phasen werden über Magnesiumsulfat getrocknet, filtriet und das Lösungsmittel abdestilliert. Das verbleibende orangefarbige Öl (14,59 g) wird in 150 ml Methanol gelöst und bei 0 °C wird eine Lösung von Oxone® (113,87 g, 185,22 mMol) in 450 ml Wasser zugetropft. Nach Angleichung des Reaktionsgemisches an Raumtemperatur wird für 3 Stunden nachgerührt. Das Reaktionsgemisch wird mit 3 x 200 ml Ether extrahiert. Die vereinigten organischen Phasen werden mit 100 ml 1 N HCL-Lösung gewaschen, über Magnesiumsulfat getrocknet, filtriert und das Lösungsmittel abdestilliert. Die verbleibenden gelben Kristalle (9,76 g) werden in Dichlormethan suspendiert. Die Kristalle werden abfiltriert und ein wenig mit kaltem Dichlormethan gewaschen.
Ausbeute: 4,80 g 5-(Phenylsulfonyl)-thiophen-2-carbonsäure (46% d. Th.) als weiße Kristalle.
Fp.: 184-185 °C.
¹H NMR (400 MHz, DMSO-*d₆*) 7.64-7.69 (m, 2 H), 7.71 (d, *J* = 4.0 Hz, 1 H), 7.72-7.77 (m, 1 H), 7.85 (d, *J =* 4.0 Hz, 1 H), 8.00-8.05 (m, 2 H). ¹³C NMR (100 MHz, DMSO-*d₆*) 127.4, 130.1, 133.4, 134.2, 134.5, 140.7, 142.2, 147.1, 161.8.

[5-(Phenylsulfonyl)-thien-2-yl]-carbamidsäure tert.-butyl ester
Zu 5-(Phenylsulfonyl)-thiophen-2-carbonsäure (1,00 g, 3.7 mMol), in 40 ml tert.-Butanol abs. werden Triethylamin (0,62 ml, 4.4 mMol) und Diphenylphosphorylazid (0,97 ml, 4,5 mMol) zugegeben. Das Reaktionsgemisch wird für 5 Stunden unter Rückfluß gekocht. Das Lösungsmittel wird abdestilliert. Der Rückstand wird aufgenommen in Ethylacetat und mit Wasser gewaschen. Die organische Phase wird über Magnesiumsulfat getrocknet, filtriert und das Lösungsmittel abdestilliert. Das verbleibende grun-schwarzes Öl wird mittels Flash-chromatographie (Eluens: 33% v/v Ethylacetat in Petrolether (40/60)) gereinigt.
Ausbeute: 520 mg [5-(Phenylsulfonyl)-thien-2-yl]-carbamidsäure *tert*.-butyl ester (41% d. Th.) als weiße Kristalle.
Fp.: 170-171°C.
¹H NMR (400 MHz, DMSO-*d₆*) 1.47 (s, 9 H), 6.53 (d, *J* = 4.3 Hz, 1 H), 7.56 d, *J* = 4.3 Hz, 1 H), 7.57-7.68 (m, 3 H), 7.87-7.92 (m, 2 H), 11.13 (bs, 1 H). ¹³C NMR (100 MHz, DMSO-*d₆*) 28.0, 81.6, 110.2, 126.5, 129.0, 129.7, 133.3 (2x C), 142.9, 150.5, 152.5.

2-Amino-5-(phenylsulfonyl)-thiophen
[5-(Phenylsulfonyl)-thien-2-yl]-carbamidsäure tert.-butyl ester (0,37 g, 1,1 mMol) wird in 5 ml Trifluoressigsäure gelöst und für 2 Stunden bei Raumtemperatur gerührt. Die Trifluoressigsäure wird abdestilliert und der Rückstand wird zwischen einer halbgesättigten Natriumhydrogencarbonat Lösung und Dichlormethan verteilt. Die Phasen werden getrennt und die wäßrige Phase mit Dichlormethan extrahiert. Die vereinigten organischen Phasen werden mit Kaliumcarbonat getrocknet, filtriert und das Lösungsmittel abdestilliert.
Ausbeute: 0,20 g 2-Amino-5-(phenylsulfonyl)-thiophen (76% d. Th.)) als leichtgelbe Kristalle.
Fp.: 144-145 °C.
¹H NMR (400 MHz, DMSO-*d₆*) 5.90 (d, *J* = 4.0 Hz, 1 H), 6.81 (bs, 2 H), 7.32 (d, *J* = 4.0 Hz, 1 H), 7.55-7.65 (m, 3 H), 7.79-7.84 (m, 2 H). ¹³C NMR (100 MHz, DMSO-*d₆*) 104.4, 117.9, 126.1, 129.5, 132.7, 136.5, 143.8, 165.1.

### Beispiel 5: N-[5-(Phenylcarbonyl)-1,3,4-thiadiazol-2-yl]-2-hydroxy-2-methylpropanamid

Zu 2-Hydroxyisobuttersäure (82.9 mg, 0.796 mMol) in 5,0 ml *N*,*N*-dimethylacetamid werden bei -20/-15 °C Thionylchlorid (60 µl, 0.82 mMol) zugegeben und bei -15/-10 °C für eine Stunde nachgerührt. Nach Zugabe von 2-Amino-5-(phenylcarbonyl)-1,3,4-thiadiazol (97.5 mg, 0.475 mMol) und Angleichung des Reaktionsgemisches an Raumtemperatur wird über Nacht nachgerührt. Das Reaktionsgemisch wird auf 75 ml Wasser gegossen und mit 3 x 25 ml Ethylacetat extrahiert. Die vereinigten organischen Phasen werden über Magnesiumsulfat getrocknet, filtriert und das Lösungsmittel abdestilliert. Das verbleibende Öl wird mittels Flash-chromatographie (Eluens: 33% v/v Ethylacetat in Petrolether (40/60)) gereinigt.
Ausbeute: 30.1 mg *N*-[5-(Phenylcarbonyl)-1,3,4-thiadiazol-2-yl]-2-hydroxy-2-methylpropanamid (22% d. Th) als leichtgelbe Kristalle.
Fp: 189-190 °C.
¹H NMR (400 MHz, CDCl₃) 1.65 (s, 6 H), 7.55-7.60 (m, 2 H), 7.68-7.73 (m, 1 H), 8.41-8.45 (m, 2 H). ¹³C NMR (100 MHz, CDCl₃) 27.6, 74.1, 128.6, 130.9, 134.2, 134.9, 163.4, 164.2, 175.7, 184.0.

### Beispiel 6: N-[5-(Phenylcarbonyl)-1,3,4-thiadiazol-2-yl]-2-hydroxy-2-methylbutanamid

Zu 2-Hydroxy-2-methylbuttersäure (96.0 mg, 0.813 mMol) in 5,0 ml *N*,*N*-dimethylacetamid werden bei -20/-15 °C Thionylchlorid (60 µl, 0.82 mMol) zugegeben und bei -15/-10 °C für eine Stunde nachgerührt. Nach Zugabe von 2-Amino-5-(phenylcarbonyl)-1,3,4-thiadiazol (102.3 mg, 0.499 mMol) und Angleichung des Reaktionsgemisches an Raumtemperatur wird über Nacht nachgerührt. Das Reaktionsgemisch wird auf 100 mL Wasser gegossen und mit 3 x 25 ml Ethylacetat extrahiert. Die vereinigten organischen Phasen werden über Magnesiumsulfat getrocknet, filtriert und das Lösungsmittel abdestilliert. Das verbleibende Öl wird mittels Flash-chromatographie (Eluens: 33% v/v Ethylacetat in Petrolether (40/60)) gereinigt.
Ausbeute: 20.0 mg *N*-[5-(Phenylcarbonyl)-1,3,4-thiadiazol-2-yl]-2-hydroxy-2-methylbutanamid (13% d. Th) als leichtgelbe Kristalle.
¹H NMR (400 MHz, CDCl₃) 0.98 (t, *J* = 7.4 Hz, 3 H), 1.61 (s, 3 H), 1.80-1.90 (m, 1 H), 2.00-2.10 (m, 1 H), 7.55-7.60 (m, 2 H), 7.68-7.73 (m, 1 H), 8.41-8.45 (m, 2 H). ¹³C NMR (100 MHz, CDCl₃) 7.8, 25.8, 33.3, 76.9, 128.6, 130.9, 134.2, 134.9, 163.3, 164.1, 175.4, 184.0.

### Beispiel 7: N-[5-(4-Methylphenylsulfonyl)-thien-2-yl]-3,3,3-trifluor-2-hydroxy-2-methylpropanamid

Zu 3,3,3-Trifluor-2-hydroxy-2-methylpropansäure (140 mg, 0,89 mMol) in 10 mL *N*,*N*-Dimethylacetamid werden bei -15/-10 °C Thionylchlorid (70 µl, 1,51 mMol) zugegeben und bei dieser Temperatur für eine Stunde nachgerührt. Anschließend wird 2-Amino-5-(4-methylphenylsulfonyl)-thiophen (145 mg, 0,57 mMol) zugegeben und das Reaktionsgemisch auf Raumtemperatur angeglichen. Nach beendeter Reaktion wird das Reaktionsgemisch auf 200 ml Wasser gegossen und mit 3 x 100 ml Ethylacetat extrahiert. Die vereinigten organischen Phasen werden über Magnesiumsulfat getrocknet, filtriert und das Lösungsmittel abdestilliert. Das verbleibende braunfarbige Öl wird mittels Flash-chromatographie (Eluens: 50% v/v Ethylacetat in Petrolether (40/60)) gereinigt.
Ausbeute 111 mg *N*-[5-(4-Methylphenylsulfonyl)-thien-2-yl]-3,3,3-trifluor-2-hydroxy-2-methylpropanamid (50% d. Th) als hellbraune Kristalle.
Fp: 191-193 °C.
¹H NMR (400 MHz, DMSO-*d₆*) 1.57 (s, 3 H), 2.37 (s, 3 H), 7.10 (d, *J* = 4.2 Hz, 1 H), 7.41 (d, *J* = 7.9 Hz, 2 H), 7.59 (bs, 1 H), 7.61 (d, *J* = 4.2 Hz, 1 H), 7.79 (d, *J* = 7.9 Hz, 2 H).

Analog wurden folgende Verbindungen hergestellt

### Beispiel 8:

### N-[5-(4-Chlorphenylsulfonyl)-thien-2-yl]-3,3,3-trifluor-2-hydroxy-2-methylpropanamid

¹H NMR (400 MHz, DMSO-*d₆*) 1.58 (s, 3 H), 7.13 (d, *J* = 4.0 Hz, 1 H), 7.66-7.70 (m, 3 H), 7.90-7.93 (m, 2 H).

### Beispiel 9: N-[5-(4-Fluorphenylsulfonyl)-thien-2-yl]-3,3,3-trifluor-2-hydroxy-2-methylpropanamid

¹H NMR (400 MHz, DMSO-*d₆*) 1.58 (s, 3 H), 7.12 (d, *J* = 4.2 Hz, 1 H), 7.41-7.46 (m, 2 H), 7.60 (s, 1 H), 7.67 (d, *J* = 4.2 Hz, 1 H), 7.96 (m, 2 H), 8.01 (m, 2 H).

### Beispiel 10: N-[5-(2-Thienylsulfonyl)-2-thienyl]-3,3,3-trifluor-2-hydroxy-2-methylpropanamid

¹H NMR (400 MHz, CDCl₃) 1.71 (s, 3 H), 6.70 (d, *J* = 4.2 Hz, 1 H), 7.03 (dd, *J* = 4.9, 3.8 Hz, 1 H), 7.50 (d, *J* = 4.2 Hz, 1 H), 7.59 (dd, *J* = 5.0, 1.3 Hz, 1 H), 7.64 (dd, *J* = 3.9, 1.3 Hz, 1 H), 9.68 (bs, 1 H).
¹³C NMR (100 MHz, CDCl₃) 20.2, 75.6 (q, ²*J*_{C,F} = 30.2 Hz), 112.9, 123.6 (q, ¹*J*_{C,F} = 285.8 Hz), 127.9, 131.4, 133.0, 133.7, 133.9, 143.7, 145.9, 165.6.

### Beispiel 11: N-[5-(2-Pyridylsulfonyl)-2-thienyl]-3,3,3-trifluor-2-hydroxy-2-methylpropanamid

¹H NMR (400 MHz, CDCl₃) 1.69 (s, 3H), 6.79 (d, *J* = 4.0 Hz), 7.43-7.47 (m, 1 H), 7.57 (d, *J* = 4.0 Hz), 7.90 (td, *J* = 8.0, 1.2 Hz, 1 H), 8.07 (d, *J* = 8.0 Hz, 1 H), 8.59-8.62 (m, 1 H), 10.11 (bs, 1 H).

### Beispiel 12: (-)-N-[5-(Phenylsulfonyl)-thien-2-yl]-3,3,3-trifluor-2-hydroxy-2-methylpropanamid

Zu *N*-[5-(Phenylsulfonyl)-thien-2-yl]-3,3,3-trifluor-2-hydroxy-2-methylpropanamid (2,00 g, 5.27 mMol) in 100 ml Dichlormethan werden bei 0°C Triethylamin (0,81 ml, 5.81 mMol) und einige Kristalle *N*,*N*-Dimethyl-4-aminopyridin zugegeben. Anschließend wird eine Lösung von (1*S*)-(-)-Camphansäurechlorid (1,26 g, 5,81 mMol) in 20 ml Dichlormethan zugetropft und das Reaktionsgemisch auf Raumtemperatur angeglichen. Nach beendeter Reaktion wird das Reaktionsgemisch auf 200 ml Wasser gegossen und mit 2 x 50 ml Dichlormethan extrahiert. Die vereinigten organischen Phasen werden über Magnesiumsulfat getrocknet, filtriert und das Lösungsmittel abdestilliert. Das Gemisch von Diastereomeren (grauer Schaum, 3,06 g) wird mittels Flash-Chromatographie (Eluens: 9 %v/v Diethylether in Dichlormethan) getrennt. Der hochlaufende Camphansäure ester wird als weißer Schaum isoliert (0,74 g, Ausbeute: 25% d. Th.)
¹H NMR (400 MHz, CDCl₃) 1.01 (s, 3H), 1.07 (s, 3H), 1.11 (s, 3H), 1.71 (ddd, *J* = 13.3, 9.3, 4.2 Hz, 1 H), 1.95 (ddd, *J* = 13.3, 10.8, 4.6 Hz, 1 H), 2.03 (s, 3 H), 2.12 (ddd, *J* = 13.6, 9.3, 4.5 Hz, 1 H), 2.43 (ddd, *J* = 13.6, 10.8, 4.2 Hz, 1 H), 6.75 (d, *J* = 4.2 Hz, 1 H), 7.45-7.57 (m, 4 H), 7.91-7.95 (m, 2 H), 9.27 (bs, 1 H). ¹³C NMR (100 MHz, CDCl₃) 9.55, 16.42, 16.50, 16.55, 28.71, 30.79, 54.58, 54.95, 81.62 (q, ²*J*_{C,F} = 30.8 Hz), 90.63, 113.38, 122.36 (q, ¹*J*_{C,F} = 285.8 Hz), 127.13, 129.32, 131.35, 133.18, 134.36, 142.34, 145.54, 160.93, 164.67, 177.61. Optische Reinheit: > 99% de (Chirale HPLC, Chiracel OD-H column, 10% v/v Ethanol in Hexane, Flußrate 1,0 ml/min).
Zum hochlaufenden Camphansäure ester (0,65 g, 1,16 mMol) in 30 ml Methanol wird bei Raumtemperatur eine 2 N NaOH-Lösung (0.60 ml, 1,2 mMol) zugegeben. Nach beendeter Reaktion wird das Lösungsmittel abdestiliert. Das verbleibende Öl wird in 250 ml Dichlormethan aufgenomen und auf 250 ml Wasser ausgegossen und mit 3 x 100 ml Dichlormethan extrahiert. Die vereinigten organischen Phasen werden über Magnesiumsulfat getrocknet, filtriert und das Lösungsmittel abdestiliert. Die verbleibenden weißen Kristalle werden aus Hexane/Ethylacetat umkristallisiert.
Ausbeute: 340 mg (-)-*N*-[5-(Phenylsulfonyl)-thien-2-yl]-3,3,3-trifluor-2-hydroxy-2-methylpropanamid (77% d. Th.) als weiße Kristalle.
Fp: 189-190°C. [α]²⁰_{D} = -46.5° (c 0.94, MeOH). Optische Reinheit: >99% ee (Chirale HPLC, Chiracel OD-H column, 10% v/v Ethanol in Hexane, Flußrate 1,0 ml/min). ¹H NMR und ¹³C NMR Data sind identisch mit dem racemischen Material.

### Beispiel A:

### Wirkungen auf die Harnblasenmuskulatur in vitro

Die Wirkung der erfindungsgemäßen Verbindungen auf die Harnblasenmuskulatur können in speziell entwickelten *in vitro* Versuchen, wie unten beschrieben, gezeigt werden. Hierbei bedeutet der IC₅₀-Wert diejenige Konzentration einer Substanz, die die Kontraktion um 50% reduziert.

Männliche Albinomeerschweinchen im Gewicht von ca. 400 g werden durch Genickschlag schmerzlos getötet und rasch entblutet. Die Bauchhöhle wird geöffnet und die Harnblase sorgfältig reseziert. Sie wird in eine mit Krebslösung gefüllte Petrischale gelegt, und umgebenes Binde- und Fettgewebe wird entfernt. Zwei horizontale Gewebestreifen werden herauspräpariert. Mithilfe zweier Fäden, die an beiden Enden der Gewebestreifen befestigt werden, werden die Streifen in ein Organbad eingehängt. Dabei werden sie unten an einem Haken und oben an einem elektrischen Kraftaufnehmer, der Kontraktionen von 0-10 g Stärke präzise messen kann, befestigt. Die gemessenen Kontraktionen werden mittels eines Brückenverstärkers verstärkt und mit einem Thermoschreiber registriert. Die erhaltenen Signale können außerdem elektronisch auf einem anderen Schreiberkanal integriert werden, um phasische Kontraktionen zu quantifizieren.

Die Organbäder werden mit 37°C Krebslösung gefüllt, die gut mit einem O₂/CO₂-Gemisch durchperlt wird und die folgende Zusammensetzung hat (mmol/l): NaCl 118.4; KCl 4.7; CaCl₂ 2.5; KH₂PO₄ 1.2; MgSO₄ 1.2; NaHCO₃ 25; Glucose 11.

Die Streifen werden mit einem Grundtonus von ca. 0.5 g gespannt. Nach einer Equilibrierzeit von ca. 30 Minuten wird einer der beiden Streifen mit eingebauten Elektroden jede Minute elektrisch gereizt (Reizdauer 2 Sekunden, 30 Hz, 0.5 mS Pulsbreite, 10 Volt), und damit kurzdauernde Kontraktionen auslöst. Der andere Streifen wird duch Depolarisation (Zugabe von 15 mM KCl ins Organbad) in phasische Kontraktionen versetzt. Sowohl die durch elektrische Reizung als auch die durch Depolarisation ausgelösten Kontraktionen der Harnblasenmuskulaturstreifen erreichen rasch ein Plateau. Sobald dieses Plateau erreicht wird ("100%-Wert"), wird die Testsubstanz ins Organbad zugegeben, zuerst in niedrigen Konzentrationen, z.B. 10⁻⁸ M, und dann in regelmässigen Intervallen in steigenden Dosen, bis die Kontraktionen sich reduzieren, oder die Endkonzentration von 10⁻⁴ M erreicht wird. Jede Testsubstanz wird so 2-4 mal getestet.

Zur Auswertung wird für jeden Versuch die Höhe der auf dem Schreiberpapier registrierten Kontraktionen nach Einwirkung von jeder zugegebenen Testkonzentration gemessen. In Bezug auf die ursprüngliche Kontraktionshöhe vor Substanzgabe (100%) läßt sich so der IC₅₀-Wert errechnen. Alle IC₅₀-Werte sind in Spalte A der Tabelle 1 dargestellt.

### Beispiel B

### Wirkungen auf die Gefäßmuskulatur in vitro

### Aortenringe

Männliche Ratten im Gewicht von ca. 300 g werden durch Genickschlag schmerzlos getötet und rasch entblutet. Die Brusthöhle wird geöffnet und die Aorta sorgfältig reseziert. Sie wird in eine mit Krebslösung gefüllte Petrischale gelegt, und umgebenes Binde- und Fettgewebe werden entfernt. Zwei Ringe werden herauspräpariert. Die Ringe werden je in ein Organbad eingehängt. Dabei werden sie unten an einem Haken und oben an einem elektrischen Kraftaufnehmer, der Kontraktionen von 0-10 Gramm Stärke präzise messen kann, befestigt. Die gemessenen Kontraktionen werden mittels eines Brückenverstärkers verstärkt und mit einem Thermoschreiber registriert.

Die Organbäder werden mit 37°C Krebslösung gefüllt, die gut mit einem O₂/CO₂-Gemisch durchperlt wird und die folgende Zusarnmensetzung hat (mmol/l): NaCl 118.4; KCl 4.7; CaCl₂ 2.5; KH₂PO₄ 1.2; MgSO₄ 1.2; NaHCO₃ 25; Glucose 11.

Die Ringe werden mit einem Grundtonus von ca. 0,5 g gespannt. Nach einer Equilibrierzeit von ca. 30 Minuten werden die Ringe duch Depolarisation (Zugabe von 25 mM KCl ins Organbad) in Kontraktur versetzt. Die durch die Depolarisation ausgelösten Kontraktionen der Aortenringen erreichen rasch ein Plateau. Sobald dieses Plateau erreicht wird ("100% Wert"), wird die Testsubstanz ins Organbad zugegeben, zuerst in niedrigen Konzentrationen, z.B. 10⁻⁸ M, und dann in regelmässigen Intervallen in steigenden Dosen, bis die Kontraktionen sich reduzieren, oder die Endkonzentration von 10⁻⁴ M erreicht wird. Jede Testsubstanz wird so 2-4 mal getestet.

Zur Auswertung wird für jeden Versuch die Höhe der auf dem Schreiberpapier registrierten Kontraktionen nach Einwirkung von jeder zugegebenen Testkonzentration gemessen. In Bezug auf die ursprüngliche Kontraktionshöhe vor Substanzgabe (100%) läßt sich so der IC₅₀-Wert errechnen. IC₅₀-Werte sind in Spalte B der Tabelle 1 dargestellt.

### Beispiel C:

### Wirkungen auf den Blutdruck in vivo

### Wache Ratte

Männlichen Sprague-Dawley Ratten (Charles River, Sulzfeld, Deutschland) im Gewicht von ca. 400g wurde vor Versuchsbeginn unter sterilen Bedingungen ein Katheter in die Arteria carotis implantiert, um den arteriellen Blutdruck mit Hilfe eines üblichen Druckaufnehmers (z.B. Statham P23d) kontinuierlich messen zu können. Den Ratten wurde über Nacht vor dem Versuchstag das Futter entzogen.
Am Versuchstag wurden die Ratten ins Versuchslabor gebracht und Blutdruck und Herzfrequenz über ca. 30 Minuten registriert. Die in 0.5% Methylcellulose suspendierten Testsubstanzen wurden dann mit einer Magensonde peroral appliziert (Volumen: 3ml/kg Körpergewicht) und Blutdruck und Herzfrequenz über die nächsten 6 Stunden gemessen. Die prozentualen Meßwertänderungen gegenüber den Ausgangswerten wurden kalkuliert und für Gruppen von 3-4 ähnlich behandelten Tieren ermittelt. Die Ergebnisse sind in Spalte C der Tabelle 1 dargestellt.

**Tabelle 1**

| Vbg | A IC₅₀ mol/l | B IC₅₀ mol/l | Selekt. | Dosis mg/kg | C Senkung d.diastolischen Drucks % des Ausgangswerts | | | |
|---|---|---|---|---|---|---|---|---|
| | | | | | 15 | 30 | 60 | 120Min |
| 1 | 1.0x10⁻⁴ | 2.5x10⁻⁶ | 0.025 | 6 | | 3 | 2 | 4 |
| 2 | 1.0x10⁻⁴ | 2.0x10⁻⁵ | 0.2 | 6 | 2 | 8 | 5 | |
| 3 | 5.0x10⁻⁵ | 1.0x10⁻⁵ | 0.2 | 6 | | 3 | 2 | 4 |
| 4 | 1.7x10⁻⁶ | 1.2x10⁻⁶ | 0.7 | 6 | 1 | 5 | 4 | 6 |
| | | | | 18 | 0 | 8 | 10 | 15 |
| V | 1.0x10⁻⁶ | 6.5x10⁻⁸ | 0.065 | 1 | 53 | 49 | 40 | 26 |

Tabelle 1: Erschlaffende Wirkung der oben genannten Substanzen auf Blasenstreifen (Spalte A) und Aortenringe (Spalte B) in vitro. Das Verhältnis IC₅₀ Blase/IC₅₀Aorta gibt die Selektivität an. In Spalte C sind die blutdrucksenkenden Wirkungen in vivo 15, 30, 60 und 120 Minuten nach peroraler Substanzgabe aufgeführt. Entsprechende Werte für die Referenzsubstanz (Verbindung V) Cromakalim werden als Vergleich dargestellt.

## Patentansprüche

1. Heterocyclische Amide der Formel I 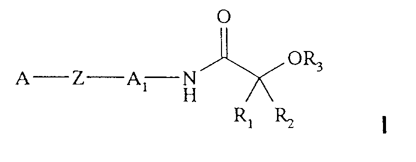 in welcher
A einen aromatischen oder S- oder N- heteroaromatischen Ring mit 5 - 10 C-Atomen, der gegebenfalls ein -oder mehrfach durch geradkettiges oder verzweigtes C₁-C₄ Alkyl oder C₁-C₄ Alkoxy, Hydroxy, Halogen oder CF₃, CF₃CF₂ substituiert sein kann,
Z die Reste C=O, SO oder SO₂,
A₁ ein heteroaromatisches System der Formel IIa-IIc 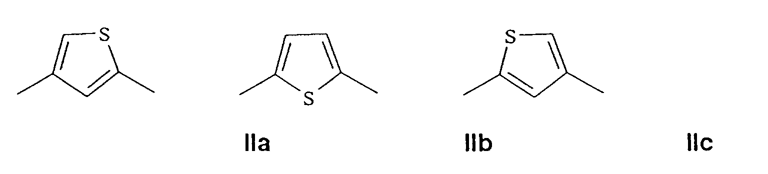 oder ein heteroaromatisches System der Formel IIIa-IIIc 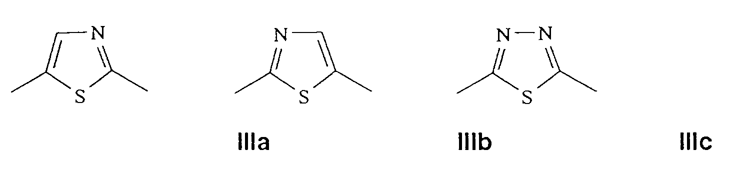 R₁ und R₂ unabhängig voneinander H, geradkettiges oder verzweigtes (C₁-C₄)-Alkyl oder fluoriertes Alkyl,
und R₃ H, geradkettiges oder verzweigtes (C₁-C₄)-Alkyl, Acetyl, Alkylacetyl oder eine unter physiologisches Bedingungen abspaltbare Gruppe bedeutet.

2. Heterocyclische Amide der Formel I nach Anspruch 1, in der
A einen gegebenenfalls durch Halogen, CF₃ oder CF₃CF₂ substituierten Phenylrest oder einen 2- oder 3- Thienylrest,
Z eine Gruppe C=O oder SO₂
A₁ eine heteroaromatisches System der Formel IIb, IIIb oder IIIc,
R₁ und R₂ unabhängig voneinander einen geradkettigen oder verzweigten Alkylrest mit 1 - 4 C- Atomen oder den Rest CF₃
und R₃ H oder einen geradkettigen oder verzweigten Alkylrest mit 1 - 4 C Atomen bedeutet.

3. Heterocyclische Amide der Formel I nach Anspruch 1, in der
A einen gegebenenfalls durch Halogen, CF₃ oder CF₃CF₂ substituierten Phenylrest oder einen 2- oder 3- Thienylrest,
Z eine Gruppe C=O oder SO₂
A₁ eine heteroaromatisches System der Formel IIb
R₁ Methyl
R₂ CF₃
und R₃ H bedeutet.

4. N-[5-(Phenylsulfonyl)-thien-2-yl]-3,3,3-trifluor-2-hydroxy-2-methylpropanamid

5. Heterocyclische Amide nach einem der Ansprüche 1 bis 4, dadurch gekennzeichnet daß die Verbindung jeweils in ihrer enantiomerenreinen Form vorliegen.

6. Heterocyclische Amide nach einem der Ansprüche 1 bis 4, dadurch gekennzeichnet, daß die Verbindungen als Mischung ihrer optischen Antipoden vorliegen.

7. Verfahren zur Herstellung von Verbindungen der Formel I 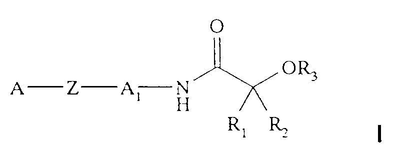 dadurch gekennzeichnet, daß man eine Verbindung der Formel IV
A - Z - A₁ - NH₂ IV
mit Hilfe bekannter Kopplungsreagenzien mit einer Verbindung der Formel **V** 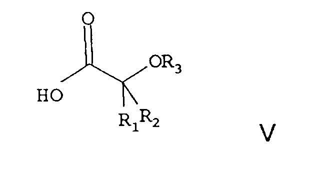 koppelt und gegebenfalls die so erhaltene Mischung der optischen Antipoden in die Enantiomeren trennt.

8. Verfahren nach Anspruch 7, dadurch gekennzeichnet, daß die Verbindung der Formel V in einer optisch aktiven Form eingesetzt wird.

9. Pharmazeutische Präparate enthaltend heterocyclische Amide der Formel I nach Anspruch 1 zusammen mit üblichen galenischen Hilfs- und/oder Trägerstoffen und/oder Verdünnungsmitteln.

10. Verwendung der heterocyclischen Amide der Formel I nach Anspruch 1 zur Behandlung von Störungen oder Krankheiten, die durch Beeinflussung von Kaliumkanälen geheilt oder gelindert werden können.

11. Verwendung der heterocyclischen Amide der Formel I nach Anspruch 1 zur Behandlung von Störungen und Krankheiten der Blase und der ableitenden Harnwege.

12. Verwendung der heterocyclischen Amide der Formel I nach Anspruch 1 zur Behandlung von Inkontinenz.
